# EUROPÄISCHE PATENTSCHRIFT

(11) **EP 1 400 589 B1**
(45) Veröffentlichungstag und Bekanntmachung des Hinweises auf die Patenterteilung: **09.08.2006**
(21) Anmeldenummer: 02020904.5
(22) Anmeldetag: 18.09.2002
(51) Int. Cl.: C12N 15/10

(54) **Verfahren zum Nachweis prokaryontischer DNA**
Method for the detection of prokaryotic DNA
Procédé de détection d'un ADN procaryote

(43) Veröffentlichungstag der Anmeldung: 24.03.2004
(73) Patentinhaber: SIRS-Lab GmbH, 07745 Jena (DE)
(72) Erfinder: Schmidt, Karl-Hermann, Dr.rer.nat.habil., 07646 Stadtroda (DE); Straube, Eberhard, Prof. Dr. med., 07745 Jena (DE); Russwurm, Stefan, Dr. med., 07743 Jena (DE)
(74) Vertreter: Geyer, Fehners & Partner

(56) Entgegenhaltungen:
- CROSS S H ET AL: "PURIFICATION OF CPG ISLANDS USING A METHYLATED DNA BINDING COLUMN" NATURE GENETICS, NEW YORK, NY, US, Bd. 6, Nr. 3, 1. März 1994 (1994-03-01), Seiten 236-244, XP000578157 ISSN: 1061-4036
- CARLONE D L ET AL: "Cloning and characterization of the gene encoding the mouse homologue of CpG binding protein" GENE: AN INTERNATIONAL JOURNAL ON GENES AND GENOMES, ELSEVIER SCIENCE PUBLISHERS, BARKING, GB, Bd. 295, Nr. 1, 24. Juli 2002 (2002-07-24), Seiten 71-77, XP004381373 ISSN: 0378-1119
- HEMMI HIROAKI ET AL: "A Toll-like receptor recognizes bacterial DNA" NATURE, MACMILLAN JOURNALS LTD. LONDON, GB, Bd. 408, Nr. 6813, 7. Dezember 2000 (2000-12-07), Seiten 740-745, XP002168474 ISSN: 0028-0836

## Beschreibung

Die Erfindung betrifft ein in vitro Verfahren zum Nachweis prokaryontischer DNA aus körperflüssigkeiten, ein Verfahren zür Reinigüng von körperflüssigkeiten von prokaryontischer DNA in vitro sowie einen Kit zur Durchführung der Verfahren.

Durch Bakterien verursachte Infektionen sind eine der häufigsten Ursachen für Entzündungskrankheiten. Zur Prognose des Krankheitsverlaufes sowie insbesondere zur rechtzeitigen Auswahl geeigneter therapeutischer Maßnahmen ist der frühzeitige Nachweis der bakteriellen Erreger von entscheidender Bedeutung.

Zum Nachweis bakterieller Erreger werden vor allen Dingen verschiedene Methoden der Kultivierung von Zellen angewendet. In letzter Zeit haben aber auch molekularbiologische Methoden, die auf dem Nachweis erregerspezifischer Nukleinsäuren basieren, an Bedeutung gewonnen. Neben der hohen Spezifität dieser Methoden ist der geringe Zeitbedarf als wesentlicher Vorteil gegenüber konventioneller Methoden zu nennen. Allerdings ist die Sensitivität des Nachweises prokaryontischer DNA direkt aus Körperflüssigkeiten und nicht vorbehandeltem Untersuchungsmaterial im Vergleich zur Kultur der Mikroorganismen bislang viel zu gering. Eine für den direkten Erregernachweis aus dem nicht vorbehandelten Untersuchungsmaterial ausreichende Menge an Nukleinsäuren von Bakterien wird allenfalls im Bereich der 16S-mRNA-Moleküle erreicht. Dies setzt aber voraus, daß sich die nachzuweisenden Bakterien in den metabolischen Phasen befinden und genügend 16S-mRNA exprimieren.

Davon ist insbesondere bei Patienten, die unter einer antibiotischen Therapie stehen, in der Regel nicht auszugehen. Darüber hinaus kommen bestimmte Pathogenitätsfaktoren von Bakterien nicht zu jeder Zeit zur Expression, obwohl die entsprechenden Gene im bakteriellen Genom vorhanden sind. Deshalb ist der Nachweis der Pathogenitätsfaktoren und Resistenzen von Bakterien auf chromosomaler Ebene für die Diagnose septischer Erkrankungen unabdingbar.

Dies umso mehr als auf dieser Ebene auch eine Unterscheidung zwischen pathogenen und kommensalen Bakterien getroffen werden kann.

Am häufigsten erfolgt der erregerspezifische Nukleinsäurenachweis durch Vervielfältigung der prokaryontischen DNA mittels der Polymerase-Kettenreaktion (PCR) bzw. der Ligase-Kettenreaktion (LCR). Der hohen Spezifität und schnellen Verfügbarkeit der Ergebnisse stehen die Störanfälligkeit durch Kontaminationen oder durch stark inhibierende Faktoren klinischer Proben gegenüber.

Bei einem herkömmlichen PCR-Nachweisverfahren ist für eine erfolgreiche Detektion von Erregern im Blut die Gesamt-DNA aus mindestens 1 bis 5 ml Blut zu isolieren. Die Gesamt-DNA-Konzentration ist dann aber zu groß, um direkt in einer PCR-Reaktion eingesetzt zu werden.

Anders verhält es sich mit der Blutkultur zum Nachweis von Erregern einer Sepsis. Dabei liegt die untere Nachweisgrenze bei weniger als 10 Bakterien pro ml. Diese Nachweisgrenze wird derzeit nur mit PCR-Protokollen erreicht, die ihre Zielsequenz im Bereich der 16S-RNA haben und damit von der Expression dieser Zielsequenz abhängig sind. Eine größere diagnostische Sicherheit ist von PCR-Protokollen zu erwarten, die ihre Zielsequenzen im Chromosom der Mikroorganismen haben. Gerade unter dem Einfluß einer laufenden Antibiotikatherapie kann das Expressionsverhalten verschiedener Gene erheblich verändert oder eingeschränkt sein, auch wenn das eingesetzte Antibiotikum letztlich nicht wirksam ist. Diese Situation ist gerade auf Intensivtherapiestationen häufig anzutreffen, auf denen die meisten Patienten unter Antibiotikatherapie stehen, bei denen aus diesem Grund keine relevanten Bakterien aus den Blutkulturen oder anderen Proben angezüchtet werden können.

Wegen unzureichender Sensitivität hat der erregerspezifische Nukleinsäurennachweis ohne Amplifikationsschritt durch direkten Nachweis der prokaryontischen DNA (Sondentechnik, FISH-Technik) nur bei ausreichend hoher Keimzahl im Untersuchungsmaterial diagnostische Bedeutung.

Die wesentliche Problematik des Nachweises prokaryontischer DNA zur Identifikation bakterieller Erreger in Körperflüssigkeiten bestehen neben PCR-hemmenden Bestandteilen im Untersuchungsmaterial vor allem in dem Überschuss an eukaroyntischer gegenüber prokaryontischen DNA. Hierbei sind insbesondere kompetitive Prozesse bei der DNA-Analyse sowie die geringe Menge an prokaryontischer DNA als hinderlich für einen qualitativen und quantitativen Erregernachweis anzusehen.

Die üblichen Methoden zur DNA-Isolierung reichern die Gesamt-DNA einer Körperflüssigkeit an, so daß das Verhältnis Wirts-DNA zu mikrobieller DNA zwischen 1 : 10⁻⁶ und 1 : 10⁻⁸ betragen kann. Aus diesem Unterschied ist die Schwierigkeit des Nachweises mikrobieller DNA in Körperflüssigkeiten gut nachzuvollziehen.

Der vorliegenden Erfindung liegt somit die Aufgabe zugrunde, eine Methode bereitzustellen, die zum Nachweis und/oder zür Anreicherung mikrobieller DNA in Untersuchungsproben mit hohem Anteil eukaryontischer DNA von Patienten mit Infektionen für einen schnellen und einfachen Erregernachweis, der eine frühzeitige Diagnose von Infektionen, durch bakterielle Erreger verursacht, ermöglicht.

Diese Aufgabe wird erfindungsgemäß gelöst durch ein Verfahren zum Nachweis prokaryontischer DNA aus Körperflüssigkeiten *in vitro* mit den Schritten
a. Kontaktierung mindestens einer in Lösung befindlichen prokaryontischen DNA mit mindestens einem Protein oder Polypeptid, das fähig ist, spezifisch an nicht-methylierte CpG-Motive zu binden,
b. Separierung des Protein/Polypeptid-DNA Komplexes aus der Lösung
c. Nachweis der prokaryontischen DNA mittels molekularbiologischer Methoden.

Die Bezeichnung prokaryontische DNA bezieht sich dabei sowohl auf virale als auch auf bakterielle DNA. Diese kann aufgereinigt und wieder in Lösung gebracht sein oder direkt in der Ursprungsquelle (z. B. Körperflüssigkeit, wie Blut, Serum, etc.) vorliegen.

Die Separation kann mittels verschiedener Verfahren zur Isolierung oder Anreicherung von DNA-Protein-Komplexen oder DNA-Polypeptid-Komplexe erfolgen, die dem Fachmann hinlänglich bekannt sind. Dabei werden bevorzugt Methoden zur Anwendung kommen, bei denen das DNA-bindende Protein an eine Trägermatrix immobilisiert ist, um die DNA aus der Probelösung anzureichern.

Gemäß einer bevorzugten Ausführungsform schließt sich an die Separation ein Schritt zur Trennung von DNA und Protein/Polypeptid an. Dies kann beispielsweise durch herkömmliche Verfahren zur DNA-Aufreinigung erfolgen, die dem Fachmann bekannt sind. Im einfachsten Falle beruht die Auftrennung auf der Änderung des pH-Wertes oder der Salzkonzentration (z. B. auf 1 M NaCl) des Mediums/Puffers oder der Zufügung chaotroper Reagenzien, etc; also geeignete Parameter, die zur Auflösung des Protein-DNA- Komplexes führen. Solche Methoden sind dem Fachmann bekannt.

Gemäß einer weiteren bevorzugten Ausführungsform, ist das Protein oder das Polypeptid an einen Träger gekoppelt. Diese Ausführungsform stellt eine besonders einfache Möglichkeit der Anreicherung prokaryontischer DNA bereit, da die Separation aus der Lösung besonders einfach, beispielsweise physikalischer Entfernung (z.B. Abzentrifugation) des oder der beladenen Träger aus der Lösung erfolgt.

Als Lösung der prokaryontischen DNA kommt grundsätzlich jedes geeignete Lösungsmittel in Frage. Besonders zweckmäßig ist das Verfahren jedoch zur Anreicherung prokaryontischer DNA aus Lösungen, die verschiedene biomolekulare Spezies, insbesondere verschieden Arten von DNA enthalten. Die Erfindung betrifft vorzugsweise ein Verfahren zum Nachweis und zur Anreicherung prokaryontischer oder viraler DNA und eukaryontischer DNA aus einem Gemisch von prokaryontischer oder viraler DNA. Dabei wird beispielsweise die in Körperflüssigkeiten befindliche prokaryontische DNA durch spezifische Bindung an das Protein oder das Polypeptid von der eukaryontischen DNA getrennt und angereichert. Die so angereicherte prokaryontische DNA erleichtert den Nachweis prokaryontischer Erreger mit Hilfe molekularbiologischer Methoden und kann zur Diagnose von Krankheiten die durch pathogene Erreger verursacht werden, beitragen.

Insbesondere die Ausführungsform, bei der das DNA-bindende Protein oder Polypeptid an die Oberfläche eines Trägers immobilisiert ist, eignet sich für eine Adsorption prokaryontischer DNA aus Körperflüssigkeiten, vorzugsweise aus dem Blut. Dieser Ansatz bietet überdies die Möglichkeit, mikrobielle DNA, die im Blut oder anderen Körperflüssigkeiten vorliegt, aus diesen zu entfernen. Die so von der mikrobiellen DNA, die auch allein in der Lage ist, schwere Entzündungsreaktionen bei Patienten auszulösen, gereinigte Körperflüssigkeit (z. B. Vollblut, Serum oder Liquor), kann dann in den Körper zurückgeführt werden.

Als Körperflüssigkeiten im Sinne der Erfindung werden alle vom Körper eines Säugers, einschließlich Mensch, stammenden Flüssigkeiten verstanden, in denen Krankheitserreger vorkommen können, wie z. B. Blut, Urin, Liquor, Pleural-, Perikardial-, Peritoneal- sowie Synovialflüssigkeit. Die auf humanes Blut bezogene Beschreibung der Erfindung stellt keine Einschränkung sondern nur eine beispielhafte Anwendung dar.

Als Proteine oder Polypeptide im Sinne der Erfindung werden alle eukaryontischen und prokaryontischen Proteine verstanden, die in der Lage sind, prokaryontische DNA spezifisch zu binden. Hierzu eignen sich insbesondere Proteine oder Polypeptide, die fähig sind, nicht-methylierte CpG-Motive spezifisch zu binden.

Unter bakteriellen Erregern werden vorzugsweise Erreger einer Sepsis, aber auch alle anderen bakteriellen Erreger von Infektionen verstanden. Sie können sich dabei von kommensalen Erregern unterscheiden, die gelegentlich auch in Untersuchungsproben von Patienten gefunden werden, aber keine pathogene Bedeutung haben.

Bei der Isolierung der Gesamt-DNA aus infizierten Körperflüssigkeiten kann das Verhältnis Wirts-DNA zur Erreger-DNA oft 1:10⁻⁶ bis 1:10⁻⁸ und weniger betragen. Das erfindungsgemäße Verfahren ermöglicht durch die spezifische Bindung prokaryontischer DNA an das Protein oder Polypeptid mit solchen selektiven Eigenschaften eine Anreicherung um 3 Potenzeinheiten und mehr.

Das Protein oder das Polypeptid kann dabei direkt oder indirekt an den Träger gekoppelt sein. Die Art der Kopplung hängt von dem Träger und dem Trägermaterial ab. Als Träger kommen dabei insbesondere Membranen, Mikropartikel und Harze oder ähnliche Materialien für Affinitätsmatrizes in Frage. Geeignete Materialien zur Anbindung des Proteins oder Polypeptides, sowie - abhängig von der Art des Materials - die Durchführung der Anbindung, sind dem Fachmann hinlänglich bekannt. Für die indirekte Kopplung eignen sich beispielsweise spezifische Antikörper gegen das Protein oder das Polypeptid, die ihrerseits durch bekannte Verfahren an den Träger gebunden sind.

Eine Anwendung des erfindungsgemäßen Verfahrens besteht im Nachweis prokaryontischer DNA. Eine weitere Anwendung besteht in der Trennung von prokaryontischer DNA aus einem Gemisch eukaryontischer und prokaryontischer DNA durch die Bindung der prokaryontischen DNA an ein spezifisches Protein oder Polypeptid, welches an eine Matrix immobilisiert wurde. Das Gemisch aus körpereigner und prokaryontischer DNA wird mittels geeigneter Verfahren mit der Affinitätsmatrix in Verbindung gebracht, und dabei wird die prokaryontische DNA an das immobilisierte Protein gebunden; die eukaryontische DNA durchläuft zum Beispiel eine Trennsäule und kann separat gesammelt werden. Affinitätsmatrizes können beispielsweise polymere Polysaccharide wie Agarosen, andere Biopolymere, synthetische Polymere, oder Träger mit Silikat-Grundgerüst wie poröse Gläser oder sonstige feste oder flexible Träger sein, an welchen das DNA-bindende Protein oder Polypeptid immobilisiert wird. Nach erfolgter Trennung prokaryontischer von eukaryontischer DNA wird die Affinitätsmatrix mit einem geeigneten Reagenz gespült, so daß entweder das Bindungsprotein mit der gekoppelten prokaryontischen DNA von der Matrix und/oder die prokaryontische DNA von dem Bindungsprotein getrennt wird und für weitere Arbeitsschritte in ausreichender Menge zur Verfügung steht.

Eine weitere Anwendung des erfindungsgemäßen Verfahrens besteht in der Trennung und Anreicherung prokaryontischer DNA von eukaryontischer DNA durch Bindung der prokaryontischen DNA an ein spezifisches Protein welches an Mikropartikeln immobilisiert wurde. Hierbei kommen alle Mikropartikel in Frage, die eine Immobilisierung des DNA-bindenden Proteins oder Polypeptids ermöglichen. Solche Mikropartikel können aus Latex, Kunststoff (z. B. Styropor, Polymer), Metall oder ferromagnetischen Stoffen bestehen. Weiterhin können auch fluoreszierende Mikropartikel, wie sie beispielsweise von der Firma Luminex angeboten werden, verwendet werden. Nachdem die prokaryontische DNA an die an Mikropartikel immobilisierten Proteine gebunden wurde, werden die Mikropartikel mit geeigneten Methoden, wie beispielsweise Filtration, Zentrifugation, Fällung, Sortierung über Messung der Fluoreszenzintensität oder magnetische Verfahren, von dem Stoffgemisch getrennt. Die prokaryontische DNA steht nach Trennung von den Mikropartikeln zur weiteren Verarbeitung zur Verfügung.

Eine andere Anwendung des erfindungsgemäßen Verfahrens besteht in der Trennung und Anreicherung prokaryontischer DNA von eukaryontischer DNA durch Bindung der prokaryontischen DNA an ein spezifisches Protein oder Polypeptid, welches anschließend durch Elektrophorese von übrigen Bestandteilen des Gemisches getrennt wird.

Eine weitere Anwendung des erfindungsgemäßen Verfahrens besteht in der Trennung und Anreicherung prokaryontischer DNA von eukaryontischer DNA durch Bindung der prokaryontischen DNA an das Protein oder Polypeptid. Dieses Protein wird anschließend an entsprechende Antikörper gebunden. Die Antikörper können an feste oder flexible Substrate, z. B. Glas, Kunststoffe, Silizium, Mikropartikel, Membranen gebunden sein, oder sich in Lösung befinden. Nach Bindung der prokaryontischen DNA an das Protein oder das Polypeptid und dessen Bindung an den spezifischen Antikörper erfolgt die Trennung aus dem Stoffgemisch mit dem Fachmann vertrauten Methoden.

Als Protein oder Polypeptid eignet sich insbesondere jedes Protein oder Polypeptid, welches prokaryontische DNA beispielsweise mit nicht-methylierten CpG-Motiven bindet. Hierzu eignen sich beispielsweise spezifische Antikörper oder Antiseren gegen prokaryontische DNA. Ihre Herstellung und Gewinnung sind dem Fachmann bekannt.

Prokaryontische DNA unterscheidet sich von eukaryontischer beispielsweise durch das Vorkommen nicht-methylierter CpG-Motive. Zweckmäßigerweise ist das Protein/Polypeptid somit ein spezifisch nicht-methylierte CpG-Motive erkennendes und bindendes Protein. Dies ist zweckmäßigerweise auch ein spezifischer Antikörper oder ein entsprechendes Antiserum. Gemäß einer weiteren bevorzugten Ausführungsform ist das Protein oder Polypeptid ein durch das TLR9-Gen oder durch das CGBP-Gen codiertes Protein oder Polypeptid.

Diese Ausführungsform der Erfindung basiert auf der Erkenntnis, daß sich eukaryontische DNA und prokaryontische DNA durch ihren Anteil an CpG-Motiven unterscheiden. In der prokaryontischen DNA befinden sich Cytosin-Guanosin-Dinukleotide (CpG-Motive) in einem 20-fachen Überschuß gegenüber eukaryontischer DNA. In prokaryontischer DNA sind diese Motive nicht-methyliert, wohingegen sie in eukaryontischer DNA zum größten Teil methyliert sind, was die Unterschiedlichkeit nochmals erhöht. Nicht-methylierte CpG-Motive sind nicht-methylierte Deoxycytidylat-Deoxyguanylat-Dinukleotide innerhalb des prokaryontischen Genoms oder innerhalb von Fragmenten desselben.

Zum zweiten basiert diese bevorzugte Ausführungsform der Erfindung auf der Erkenntnis, daß es Proteine oder Polypeptide gibt, die spezifisch an nicht-methylierte CpG-Motive der DNA binden. Die Bindungseigenschaft dieser Proteine/Polypeptide wird erfindungsgemäß genutzt, um prokaryontische DNA einerseits zu binden und damit andererseits aus einer Probe mit überwiegendem Anteil eukaryontischer DNA anzureichern.

Eine Anwendung zur Isolierung von cDNA, welche das Vorkommen methylierter CpG-Motive in eukaryontischer DNA nutzt, wurde von Cross et al. Nature Genetics 6 (1994) 236-244 beschrieben. Die immunstimulatorische Anwendung von einzelsträngigen Oligodesoxribonukleotiden (ODN) mit den ent-sprechenden CpG-Motiven konnte mehrfach gezeigt werden (Häcker et al., Immunology 105 (2002) 245-251, US 6,239,116). Als Erkennungsmoleküle der prokaryontischen CpG-Motive wurden bisher zwei Rezeptorproteine identifiziert. Aus der WO 02/06482 ist der Toll-like-Rezeptor 9 als Erkennungsmolekül nicht-methylierter CpG-Motive bekannt. Voo et al. Molecular and Cellular Biology (2000) 2108-2121 beschreiben ein weiteres Rezeptorprotein, das humane CpG bindende Protein (hCGBP), das in einem analytischen Ansatz als Erkennungsmolekül zum Nachweis von nicht-methylierten CpG-Motiven in prokaryontischer DNA verwendet wird. In beiden Publikationen werden die CpGbindenden Proteine nicht zur Isolierung oder Anreicherung prokaryontischer DNA genutzt.

Besonders geeignet ist ein Protein oder Polypeptid, welches durch eine cDNA einer Sequenz mit mindestens 80 %, vorzugsweise mindestens 90 % und besonders bevorzugt mindestens 95 %, Homologie zur Sequenz gemäß der GenBank Zugangs-Nr.: NM-014593 (Version NM-014593 1, GI: 7656974; NCBI Datenbank) codiert wird. Hierbei handelt es sich um Proteine oder Polypeptide, die dem CGBP entsprechen oder davon abgeleitet sind und CpG-Motive spezifisch erkennen und binden.

Gemäß einer weiteren bevorzugten Ausführungsform wird das Protein oder Polypeptid durch eine cDNA einer Sequenz mit mindestens 80 %, vorzugsweise mindestens 90 % Homologie zur Sequenz gemäß der GenBank Zugangs-Nr. AB045180 (kodierende Sequenz des TLR9-Gens; NCBI Datenbank, Version AB045180.1;GI: 11761320) oder ein Fragment davon, vorzugsweise cDNA mit mindestens 80 %, besonders bevorzugt 90 %, Homologie zu Transkript Variante A (GenBank Zugangs-Nr. NM-017442, Version NM-017442.2; GI: 20302169; NCBI Datenbank) oder Transkript Variante B (GenBank Zugangs-Nr. NM-138688; Version NM-138688.1; GI: 20302170; NCBI-Datenbank) codiert.

Die Erfindung betrifft überdies ein Verfahren zur Reinigung von Körperflüssigkeiten von prokaryontischer DNA in vitro.

Dieses Verfahren umfaßt die Schritte
a. Kontaktierung mindestens einer in einer Körperflüssigkeit befindlichen prokaryontischen DNA mit mindestens einem Protein oder Polypeptid, das fähig ist, spezifisch an nicht-methylierte CpG-Motive zu binden,
b. Separierung des Protein/Polypeptid-DNA Komplexes aus der Körperflüssigkeit.

Hierbei ist es zweckmäßig, daß die Separation extrakorporal unter sterilen Bedingungen erfolgt, damit die Körperflüssigkeiten wieder in den Körper zurückgeführt werden können, so daß das körpereigene Immunsystem bei der Beseitigung von Infektionen unterstützt wird, indem die sich in den Körperflüssigkeiten befindende prokaryontische DNA entfernt wird.

Bei der extrakorporalen Entfernung der prokaryontischen DNA aus Körperflüssigkeiten kommen alle geeigneten chemischen, mechanischen oder elektrochemischen Verfahren in Betracht. Weiterhin stellt auch die Kombination mit anderen extrakorporalen therapeutischen Verfahren, wie Hämoperfusion, Herz-Lungen-Maschine oder Endotoxin-Adsorber, eine weitere zweckmäßige Anwendung dar. Die Aufzählung stellt keine Begrenzung der Verfahren dar.

Gemäß einer besonders bevorzugten Ausführungsform betrifft die Erfindung ein Verfahren zur Detektion prokaryontischer DNA. Hierbei schließt sich nach der Anreicherung der prokaryontischen DNA ein Schritt zur Amplifikation der prokaryontischen DNA an, wozu sich alle gängigen Amplifikationsmethoden eignen (PCR, LCR;LM-PCR, etc.).

Die Erfindung betrifft darüber hinaus die Verwendung eines Kits zur Anreicherung prokaryontischer DNA mittels eines der vorstehend beschriebenen Verfahren, in dem zumindest das Protein/Polypeptid, vorzugsweise weitere geeignete Reagenzien zur Verfahrensdurchführung enthalten sind.

Gemäß einer bevorzugten Ausführungsform enthält der Kit neben dem Protein/Polypeptid mindestens ein Set von Primern, welche zur Amplifikation genomischer DNA bestimmter Prokaryonten unter Standardbedingungen geeignet sind.

Die Erfindung hat den Vorteil, daß durch spezifische Bindung nicht-methylierter CpGmotivreicher prokaryontischer DNA an Proteine mit spezifischer Affinität für solche Strukturen eine Konzentrierung prokaryontischer DNA aus der Gesamt-DNA eines infizierten Wirts gelingt und damit die Nachweisempfindlichkeit von Erreger-DNA in Körperflüssigkeiten stark erhöht wird.

Die Abtrennungsmöglichkeiten prokaryontischer DNA von eukaryontischer DNA mit einem spezifisch bindenden Protein sind nicht zeitaufwendiger als bekannte Methoden zur Isolierung von Gesamt-DNA. Der nachfolgende Nachweis kann aber nur über eine PCR-Reaktion erfolgen. Eine nested PCR wird in den meisten Fällen nicht notwendig sein, so daß eine beträchtliche Zeitersparnis in der Diagnostik möglich wird.

Die Erfindung soll nachfolgend anhand von Beispielen näher erläutert werden ohne sie darauf einzuschränken.
Abb. 1 zeigt die PCR von Streptokokken-DNA im Humanblut, und
Abb. 2 zeigt die nested-PCR mit den PCR-Produkten nach Abb.1.

### Beispiel 1: Nachweisverfahren nach dem Stand der Technik.

Frisches, heparinisiertes Humanblut, das Streptococcus pyogenes mit 103/ml koloniebildende Einheiten als Erreger enthält, wird für den Erregernachweis verwendet. Die DNA wird mittels Absorption an DNA bindende Matrix mit kommerziellen Kits zur Isolierung von Gesamt-DNA aus Körperflüssigkeiten nach abgewandelter Vorschrift der Hersteller isoliert. Dazu werden 100 µl infiziertes Blut in Eppendorf Tubes mit 200 µl des gesamten Lysispuffer, der Proteinase K und SDS enthält, versetzt. Das Gemisch wird 30 min bei 37°C inkubiert, und danach 20 min auf 95°C erhitzt. Nach dem Abkühlen werden 20µg Mutanolysin zugegeben und weitere 60 min bei 37°C inkubiert. Nach Zentrifugation wird der Überstand auf die Zentri-fugationssäulen mit DNA-bindender Matrix aufgetragen und die DNA nach Vorschrift des Herstellers gereinigt. Die gereinigte DNA wird in einem Endvolumen von 100µl 0.01 molar Trispuffer, pH 7.5 oder in gleicher Menge Elutionpuffer des Herstellers aufgenommen. Für den Erregernachweis werden Primer zur Identifizierung des Streptolysin O Gens(slo) ausge-wählt.

### 1. PCR. Amplifikation eines 465 bp Fragmentes

Forward-Primer 1: 5'-AGCATACAAGCAAATTTTTTACACCG
Reverse-Primer 2: 5'-GTTCTGTTATTGACACCCGCAATT
Primer Konzentration 1 mg/ml
Ansatz: 5 µl DNA-Isolat
0.5 µl Primer fw 1
0.5 µl Primer rv 2
14 µl Aqua dest
total 25 µl in Ready to go Kit (Amersham-Biosciences)

| | | |
|---|---|---|
| Reaktion: | | |
| | 1 x | 5 min 95°C |
| | 40 Zyklen à | 30 sec. 95°C |
| | | 30 sec. 51°C |
| | | 3 min 72°C |
| | 1 x | 7 min 72°C |

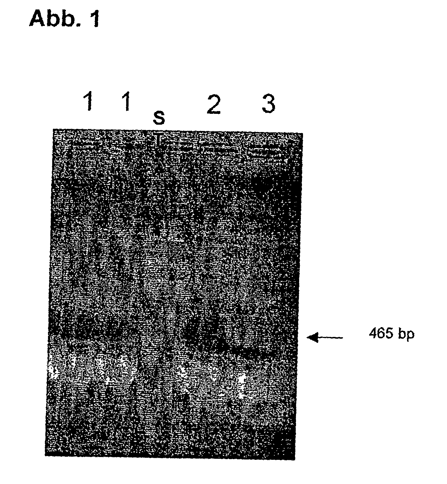

Die Ergebnisse der PCR von Streptokokken-DNA in Humanblut sind in Abb. 1 dargestellt. Es wurden 10 µl des 25 µl Ansatzes aufgetrennt: 1) PCR Ansatz mit 5 µl Template DNA; 2) Ansatz mit 5 µl Template, 1 :10 verdünnt. 3) Positivkontrolle: 0.2 µl Streptokokken-DNA als Template ohne Anwesenheit eukaryontischer DNA aus Blut. ST) Molekulargewichtsstandard

Ergebnis: Die Primär-PCR ergibt kein sichtbares PCR-Produkt. Deshalb wurde nachfolgend eine 2. PCR (nested PCR) durchgeführt.

### 2. PCR (nested): Amplifikation eines innerhalb des obigen slo-Fragmentes 348 bp Fragmentes

Forward Primer 3: 5'- CCTTCCTAATAATCCTGCGGATGT-3'
Reverse Primer 4: 5'- CTGAAGGTAGCATTAG TCTTTGATAACG-3'
Primer-Konzentration: 1mg/ml
Ansatz: 5 µl aus PCR1, Probe 1, Abb. 1
0.5 µl Primer fw 1
0.5 µl Primer rv 2
14 µl Aqua dest
total 25 µl in Ready to go Kit (Amersham-Biosciences)

| | | |
|---|---|---|
| Reaktion: | | |
| | 1 x | 5 min 95°C |
| | 50 Zyklen à | 30 sec. 95°C |
| | | 30 sec. 54°C |
| | | 3 min 72°C |
| | 1 x | 7 min 72°C |

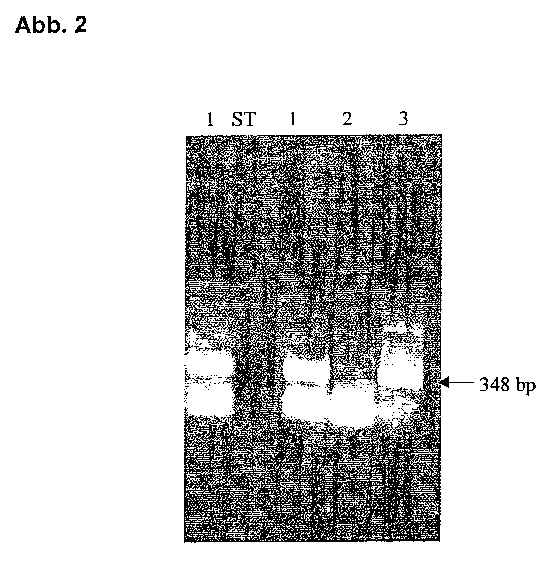

Abb. 2 zeigt die Nested PCR mit den PCR-Produkten aus dem 1. PCR-Ansatz nach Abb. 1 als Template. Die Proben entsprechen denen aus Abb. 1.

Ergebnis: In der nested PCR wird das gewünschte slo-DNA Fragment amplifiziert bei einer Erregerzahl von 100 Streptokokkenzellen pro 100 µl Blut (Probe 1). Das entspricht bei 5 µl Template DNA in der 1. PCR (Abb.1) ca. 5 bis 10 Template-Molekülen. Bei einer 1 : 10 Verdünnung (Probe 2) ist die Empfindlichkeit erschöpft (0,5 bis 1 Template-Molekülen).

### Beispiel 2: Durchführung des erfindungsgemäßen Verfahrens

Die DNA wird aus einem Zelllysat wie oben für die bisherigen PCR-Verfahren beschrieben in Lösung gebracht. Der Unterschied ist, daß zwischen 1 ml und 5 ml Untersuchungsmaterial eingesetzt werden.

Drei Milliliter frisches, heparinisiertes oder Citrat-versetztes Humanblut, das Streptococcus pyogenes mit 102/ml koloniebildende Einheiten als Erreger enthält wird für den Erregernachweis verwendet. Die DNA wird mit Lysepuffern, die SDS und Proteinase K enthalten, aus kommerziellen Kits zur Isolierung von Gesamt-DNA aus Körperflüssigkeiten nach abgewandelter Vorschrift der Hersteller isoliert. Dazu werden 3 ml infiziertes Blut mit 6 ml des gesamten Lysispuffer, der Proteinase K und SDS enthält, versetzt. Das Gemisch wird 30 min bei 37°C inkubiert, und danach 20 min auf 95°C erhitzt. Nach dem Abkühlen werden 200 µg Mutanolysin zugegeben und weitere 60 min bei 37 °C inkubiert. Nach Zentrifugation wird das Gemisch mit Ethanol bei einer Endkonzentration von 70 % gefällt und nach Zentrifugation wird das Pellet mit 2 ml 70 %igem Ethanol gewaschen. Der Ethanolrest wird in einer Vakuumzentrifuge entfernt und die gefällte DNA in 500 µl TE-Puffer aufgenommen. Die DNA wird dann auf eine Säule, die 0.5 ml Sepharose enthält und an die 1 mg TLR9 immobilisiert ist, aufgebracht. Die Säule wird mit 5 Volumen TE-Puffer gewaschen. Die Elution erfolgt mit chaotropen Ionen in hoher Konzentration, z. B. mit 0,7 ml 6 molarer NaJ oder KSCN Lösung. Dieses Eluat kann dann direkt auf eine kommerzielle DNA-Isolierungszentrifugationssäule aufgetragen werden und die CpG-angereicherte DNA nach Vorschrift, wie im Eingangsbeispiel, auf ein kleines Volumen zwischen 20 µl und 100µl isoliert und zur weiteren Analyse, wie Erreger-PCR, eingesetzt werden.

## Patentansprüche

1. Verfahren zum Nachweis prokaryontischer DNA aus Körperflüssigkeiten in vitro mit den Schritten
a. Kontaktierung mindestens einer in Lösung befindlichen prokaryontischen DNA mit mindestens einem Protein oder Polypeptid, das fähig ist, spezifisch an nicht-methylierte CpG-Motive zu binden,
b. Separierung des Protein/Polypeptid-DNA Komplexes aus der Lösung
c. Nachweis der prokaryontischen DNA mittels molekularbiologischer Methoden.

2. Verfahren gemäß Anspruch 1, **dadurch gekennzeichnet, dass** sich an die Separierung ein Schritt zur Trennung von DNA und Protein/Polypeptid anschließt.

3. Verfahren gemäß einem der vorstehenden Ansprüche, **dadurch gekennzeichnet, dass** die prokaryontische DNA mittels Amplifikation nachgewiesen wird.

4. Verfahren gemäß einem der vorstehenden Ansprüche, **dadurch gekennzeichnet, dass** die prokaryontische DNA mittels Sondentechnik nachgewiesen wird.

5. Verfahren zur Reinigung von Körperflüssigkeiten von prokaryontischer DNA *in vitro* mit den Schritten
a. Kontaktierung mindestens einer in einer Körperflüssigkeit befindlichen prokaryontischen DNA mit mindestens einem Protein oder Polypeptid, das fähig ist, spezifisch an nicht-methylierte CpG-Motive zu binden,
b. Separierung des Protein/Polypeptid-DNA Komplexes aus der Körperflüssigkeit.

6. Verfahren gemäß einem der vorstehenden Ansprüche, **dadurch gekennzeichnet, dass** das Protein/Polypeptid an einen Träger gekoppelt ist.

7. Verfahren gemäß Anspruch 6, **dadurch gekennzeichnet, dass** das Protein/Polypeptid direkt an den Träger gekoppelt ist.

8. Verfahren gemäß Anspruch 6, **dadurch gekennzeichnet, dass** das Protein/Polypeptid über einen Antikörper an den Träger gekoppelt ist.

9. Verfahren gemäß einem der Ansprüche 6 bis 8, **dadurch gekennzeichnet, dass** der Träger als Matrix, Mikropartikel oder Membran ausgebildet ist.

10. Verfahren gemäß einem der Ansprüche 1 oder 2, **dadurch gekennzeichnet, dass** die Separierung mittels eines gegen das Protein/Polypeptid gerichteten Antikörpers oder Antiserums erfolgt.

11. Verfahren gemäß Anspruch 1, **dadurch gekennzeichnet, dass** die Separierung mittels Elektrophorese erfolgt.

12. Verfahren gemäß einem der vorstehenden Ansprüche, **dadurch gekennzeichnet, dass** das Protein/Polypeptid ein gegen unmethylierte CpG-Motive gerichteter Antikörper oder ein entsprechendes Antiserum ist.

13. Verfahren gemäß einem der Ansprüche 1 bis 11, **dadurch gekennzeichnet, dass** das Protein/Polypeptid durch das TLR9 Gen oder durch das CGBP Gen codiert wird.

14. Verfahren gemäß Anspruch 13, **dadurch gekennzeichnet, dass** das Protein/Polypeptid durch eine cDNA einer Sequenz mit mindestens 80%, vorzugsweise mindestens 90% Homologie zur folgenden Sequenz kodiert wird und fähig ist, spezifisch an nicht-methylierte CpG-Motive zu binden.

15. Verfahren gemäß Anspruch 13, **dadurch gekennzeichnet, dass** das Protein/Polypeptid durch eine cDNA einer Sequenz mit mindestens 80 %, vorzugsweise mindestens 90% Homologie zur Sequenz gemäß der folgenden Sequenz oder einem Fragment davon, vorzugsweise einer cDNA mit mindestens 80 %, besonders bevorzugt mindestens 90% Homologie zu Transkript Variante A der folgenden Sequenz oder Transkript Variante B der folgenden Sequenz kodiert wird und fähig ist, spezifisch an nicht-methylierte CpG-Motive zu binden.

16. Verfahren zur Reinigung von Körperflüssigkeiten von prokaryontischer DNA gemäß Anspruch 5, **dadurch gekennzeichnet, dass** die Separierung extrakorporal unter sterilen Bedingungen erfolgt.

17. Verwendung eines Kits, enthaltend mindestens ein Protein oder Polypeptid, das fähig ist, spezifisch an nicht-methylierte CpG-Motive zu binden, zur Anreicherung prokaryontischer DNA mittels eines Verfahrens nach einem der Ansprüche 1, 2 oder 4 bis 15.

18. Verwendung eines Test-Kits, enthaltend mindestens ein Protein oder Polypeptid, das fähig ist, spezifisch an nicht-methylierte CpG-Motive zu binden und einen oder mehrere Set(s) spezifischer Primer, zur Detektion prokaryontischer DNA mittels eines Verfahrens nach Anspruch 15.

## Claims

1. Method for detecting prokaryotic DNA from body fluids in vitro, comprising the steps of:
a) contacting at least one prokaryotic DNA in solution with at least one protein or polypeptide which is capable of specifically binding to non-methylated CpG motifs,
b) separating the protein/polypeptide-DNA complex from the solution,
c) detecting the prokaryotic DNA by means of methods of molecular biology.

2. Method according to claim 1, **characterised in that** the separation is followed by a step of separating the DNA and the protein/polypeptide.

3. Method according to one of the preceding claims, **characterised in that** the prokaryotic DNA is detected by means of amplification.

4. Method according to one of the preceding claims, **characterised in that** the prokaryotic DNA is detected by means of probe technology.

5. Method for purifying body fluids from prokaryotic DNA in vitro comprising the steps of:
a) contacting at least one prokaryotic DNA in a body fluid with at least one protein or polypeptide which is capable of specifically binding to non-methylated CpG motifs,
b) separating the protein/polypeptide-DNA complex from the body fluid.

6. Method according to one of the preceding claims, **characterised in that** the protein/polypeptide is coupled to a carrier.

7. Method according to claim 6, **characterised in that** the protein/polypeptide is directly coupled to a carrier.

8. Method according to claim 6, **characterised in that** the protein/polypeptide is coupled to the carrier via an antibody.

9. Method according to one of the claims 6 to 8, **characterised in that** the carrier is provided as a matrix, microparticles or a membrane.

10. Method according to one of claims 1 or 2, **characterised in that** the separation takes place by means of an antibody or antiserum directed against the protein/polypeptide.

11. Method according to claim 1, **characterised in that** the separation is carried out by means of electrophoresis.

12. Method according to one of the preceding claims, **characterised in that** the protein/polypeptide is an antibody directed against non-methylated CpG motifs, or is a corresponding antiserum.

13. Method according to one of the claims 1 to 11, **characterised in that** the protein/polypeptide is encoded by the TLR9 gene or by the CGBP gene.

14. Method according to claim 13, **characterised in that** the protein/polypeptide is encoded by cDNA with a sequence which is at least 80% and preferably at least 90% homologous to the following sequence and is capable of specifically binding to non-methylated CpG motifs.

15. Method according to claim 13, **characterised in that** the protein/polypeptide is encoded by cDNA with a sequence which is at least 80% and preferably at least 90% homologous to the following sequence or a fragment thereof, preferably cDNA which is at least 80% and preferably at least 90% homologous to transcript variant A of the following sequence or transcript variant B of the following sequence and is capable of specifically binding to non-methylated CpG motifs.

16. Method for purifying body fluids from prokaryotic DNA according to claim 5, **characterised in that** the separation takes place extracorporally under sterile conditions.

17. Use of a kit, containing at least one protein or polypeptide which is capable of specifically binding to non-methylated CpG motifs for enriching prokaryotic DNA by means of a method according to one of the claims 1, 2 or 4 to 15.

18. Use of a test kit containing at least one protein or polypeptide which is capable of specifically binding to non-methylated CpG motifs and has one or more sets of specific primers for detecting prokaryotic DNA by a method according to claim 15.

## Revendications

1. Procédé de détection *in vitro* d'ADN procaryote provenant de liquides corporels, comprenant les étapes consistant à
a. mettre en contact au moins un ADN procaryote se trouvant en solution avec au moins une protéine ou un polypeptide qui est capable de se lier spécifiquement à des motifs CpG non méthylés,
b. séparer le complexe protéine/polypeptide-ADN de la solution
c. mettre en évidence l'ADN procaryote aux moyens de procédés de biologie moléculaire.

2. Procédé selon la revendication 1, **caractérisé en ce qu'**il s'adjoint à la séparation, une étape de séparation de l'ADN et de la protéine/du polypeptide.

3. Procédé selon l'une quelconque des revendications précédentes, **caractérisé en ce que** l'ADN procaryote est détecté par amplification.

4. Procédé selon l'une quelconque des revendications précédentes, **caractérisé en ce que** l'ADN procaryote est détecté par une technique de sondes.

5. Procédé d'épuration *in vitro* de liquides corporels, de l'ADN procaryote, comprenant les étapes consistant à
a. mettre en contact au moins un ADN procaryote se trouvant dans un liquide corporel, avec au moins une protéine ou un polypeptide qui est capable de se lier spécifiquement à des motifs CpG non méthylés,
b. séparer le complexe protéine/polypeptide-ADN du liquide corporel.

6. Procédé selon l'une quelconque des revendications précédentes, **caractérisé en ce que** la protéine/le polypeptide est couplé à un support.

7. Procédé selon la revendication 6, **caractérisé en ce que** la protéine/le polypeptide est couplé directement au support.

8. Procédé selon la revendication 6, **caractérisé en ce que** la protéine/le polypeptide est couplé au support par le biais d'un anticorps.

9. Procédé selon l'une des revendications 6 à 8, **caractérisé en ce que** le support est constitué en tant que matrice, microparticules ou membrane.

10. Procédé selon l'une des revendications 1 ou 2, **caractérisé en ce que** la séparation s'effectue au moyen d'un anticorps ou d'un antisérum dirigé contre la protéine/le polypeptide.

11. Procédé selon la revendication 1, **caractérisé en ce que** la séparation s'effectue par électrophorèse.

12. Procédé selon l'une quelconque des revendications précédentes, **caractérisé en ce que** la protéine/le polypeptide est un anticorps ou un antisérum correspondant, dirigé contre des motifs CpG non méthylés.

13. Procédé selon l'une des revendications 1 à 11, **caractérisé en ce que** la protéine/le polyptpide est codé par le gène TLR9 ou par le gène CGPB.

14. Procédé selon la revendication 13, **caractérisé en ce que** la protéine/le polypeptide est codé par un ADNc d'une séquence comportant une homologie d'au moins 80 %, de préférence d'au moins 90 % avec la séquence suivante et peut se lier spécifiquement à des motifs CpG non méthylés.

15. Procédé selon la revendication 13, **caractérisé en ce que** la protéine/le polypepytide est codé par un ADNc d'une séquence présentant une homologie d'au moins 80 %, de préférence d'au moins 90 % avec la séquence selon la séquence suivante ou un fragment de celui-ci, de préférence un ADNc présentant une homologie d'au moins 80 %, de manière particulièrement préférée, d'au moins 90 % avec la variante A de transcription de la séquence suivante ou la variante B de transcription de la séquence suivante et peut se lier de manière spécifique à des motifs CpG non méthylés.

16. Procédé d'épuration de liquides corporels, de l'ADN procaryote selon la revendication 5, **caractérisé en ce que** la séparation s'effectue de manière extracorporelle dans des conditions stériles.

17. Utilisation de trousses contenant au moins une protéine ou un polypeptide pouvant se lier de manière spécifique à des motifs CpG non méthylés, pour enrichir un ADN procaryote, par un procédé selon l'une quelconque des revendications 1, 2 ou 4 à 15.

18. Utilisation d'une trousse de test, contenant au moins une protéine ou un polypeptide capable de se lier de manière spécifique à des motifs CpG non méthylés et un ou plusieurs jeux d'amorces spécifiques, pour la détection d'ADN procaryote au moyen d'un procédé selon la revendication 15.


### SEQUENZPROTOKOLL

<110> SIRS-Lab GmbH
<120> Verfahren zum Nachweis prokaryontischer DNA
<130> Pat3696/25-EP
<140> 02 020 904.5
   <141> 2002-09-18
<160> 8
<170> PatentIn Ver. 2.1
<210> 1
   <211> 26
   <212> DNA
   <213> Künstliche Sequenz
<220>
   <223> Beschreibung der künstlichen Sequenz:Primer
<400> 1
   agcatacaag caaatttttt acaccg 26
<210> 2
   <211> 24
   <212> DNA
   <213> Künstliche Sequenz
<220>
   <223> Beschreibung der künstlichen Sequenz:Primer
<400> 2
   gttctgttat tgacacccgc aatt 24
<210> 3
   <211> 24
   <212> DNA
   <213> Künstliche Sequenz
<220>
   <223> Beschreibung der künstlichen Sequenz:Primer
<400> 3
   ccttcctaat aatcctgcgg atgt 24
<210> 4
   <211> 28
   <212> DNA
   <213> Künstliche Sequenz
<220>
   <223> Beschreibung der künstlichen Sequenz:Primer
<400> 4
   ctgaaggtag cattagtctt tgataacg 28
<210> 5
   <211> 2452
   <212> DNA
   <213> Homo sapiens
<220>
   <223> CpG binding protein (CGBP)
<400> 5
   1 ggcacgagag atggcggcgc ctgaggggtc ttgggggctc taggccggcc acctactggt
   61 ttgcagcgga gacgacgcat ggggcctgcg caataggagt acgctgcctg ggaggcgtga
   121 ctagaagcgg aagtagttgt gggcgccttt gcaaccgcct gggacgccgc cgagtggtct
   181 gtgcaggttc gcgggtcgct ggcgggggtc gtgagggagt gcgccgggag cggagatatg
   241 gagggagatg gttcagaccc agagcctcca gatgccgggg aggacagcaa gtccgagaat
   301 ggggagaatg cgcccatcta ctgcatctgc cgcaaaccgg acatcaactg cttcatgatc
   361 gggtgtgaca actgcaatga gtggttccat ggggactgca tccggatcac tgagaagatg
   421 gccaaggcca tccgggagtg gtactgtcgg gagtgcagag agaaagaccc caagctagag
   481 attcgctatc ggcacaagaa gtcacgggag cgggatggca atgagcggga cagcagtgag
   541 ccccgggatg agggtggagg gcgcaagagg cctgtccctg atccaaacct gcagcgccgg
   601 gcagggtcag ggacaggggt tggggccatg cttgctcggg gctctgcttc gccccacaaa
   661 tcctctccgc agcccttggt ggccacaccc agccagcatc accagcagca gcagcagcag
   721 atcaaacggt cagcccgcat gtgtggtgag tgtgaggcat gtcggcgcac tgaggactgt
   781 ggtcactgtg atttctgtcg ggacatgaag aagttcgggg gccccaacaa gatccggcag
   841 aagtgccggc tgcgccagtg ccagctgcgg gcccgggaat cgtacaagta cttcccttcc
   901 tcgctctcac cagtgacgcc ctcagagtcc ctgccaaggc cccgccggcc actgcccacc
   961 caacagcagc cacagccatc acagaagtta gggcgcatcc gtgaagatga gggggcagtg
   1021 gcgtcatcaa cagtcaagga gcctcctgag gctacagcca cacctgagcc actctcagat
   1081 gaggacctac ctctggatcc tgacctgtat caggacttct gtgcaggggc ctttgatgac
   1141 aatggcctgc cctggatgag cgacacagaa gagtccccat tcctggaccc cgcgctgcgg
   1201 aagagggcag tgaaagtgaa gcatgtgaag cgtcgggaga agaagtctga gaagaagaag
   1261 gaggagcgat acaagcggca tcggcagaag cagaagcaca aggataaatg gaaacaccca
   1321 gagagggctg atgccaagga ccctgcgtca ctgccccagt gcctggggcc cggctgtgtg
   1381 cgccccgccc agcccagctc caagtattgc tcagatgact gtggcatgaa gctggcagcc
   1441 aaccgcatct acgagatcct cccccagcgc atccagcagt ggcagcagag cccttgcatt
   1501 gctgaagagc acggcaagaa gctgctcgaa cgcattcgcc gagagcagca gagtgcccgc
   1561 acccgccttc aggaaatgga acgccgattc catgagcttg aggccatcat tctacgtgcc
   1621 aagcagcagg ctgtgcgcga ggatgaggag agcaacgagg gtgacagtga tgacacagac
   1681 ctgcagatct tctgtgtttc ctgtgggcac cccatcaacc cacgtgttgc cttgcgccac
   1741 atggagcgct gctacgccaa gtatgagagc cagacgtcct ttgggtccat gtaccccaca
   1801 cgcattgaag gggccacacg actcttctgt gatgtgtata atcctcagag caaaacatac
   1861 tgtaagcggc tccaggtgct gtgccccgag cactcacggg accccaaagt gccagctgac
   1921 gaggtatgcg ggtgccccct tgtacgtgat gtctttgagc tcacgggtga cttctgccgc
   1981 ctgcccaagc gccagtgcaa tcgccattac tgctgggaga agctgcggcg tgcggaagtg
   2041 gacttggagc gcgtgcgtgt gtggtacaag ctggacgagc tgtttgagca ggagcgcaat
   2101 gtgcgcacag ccatgacaaa ccgcgcggga ttgctggccc tgatgctgca ccagacgatc
   2161 cagcacgatc ccctcactac cgacctgcgc tccagtgccg accgctgagc ctcctggccc
   2221 ggacccctta aaccctgcat tccagatggg ggagccgccc ggtgcccgtg tgtccgttcc
   2281 tccactcatc tgtttctccg gttctccctg tgcccatcca ccggttgacc gcccatctgc
   2341 ctttatcaga gggactgtcc ccgtcgacat gttcagtgcc tggtggggct gcggagtcca
   2401 ctcatccttg cctcctctcc ctgggttttg ttaataaaat tttgaagaaa cc
<210> 6
   <211> 3257
   <212> DNA
   <213> Homo sapiens
<220>
   <223> Transkript Variante A des TLR9-Gens
<400> 6
   1 ccgctgctgc ccctgtggga agggacctcg agtgtgaagc atccttccct gtagctgctg
   61 tccagtctgc ccgccagacc ctctggagaa gcccctgccc cccagcatgg gtttctgccg
   121 cagcgccctg cacccgctgt ctctcctggt gcaggccatc atgctggcca tgaccctggc
   181 cctgggtacc ttgcctgcct tcctaccctg tgagctccag ccccacggcc tggtgaactg
   241 caactggctg ttcctgaagt ctgtgcccca cttctccatg gcagcacccc gtggcaatgt
   301 caccagcctt tccttgtcct ccaaccgcat ccaccacctc catgattctg actttgccca
   361 cctgcccagc ctgcggcatc tcaacctcaa gtggaactgc ccgccggttg gcctcagccc
   421 catgcacttc ccctgccaca tgaccatcga gcccagcacc ttcttggctg tgcccaccct
   481 ggaagagcta aacctgagct acaacaacat catgactgtg cctgcgctgc ccaaatccct
   541 catatccctg tccctcagcc ataccaacat cctgatgcta gactctgcca gcctcgccgg
   601 cctgcatgcc ctgcgcttcc tattcatgga cggcaactgt tattacaaga acccctgcag
   661 gcaggcactg gaggtggccc cgggtgccct ccttggcctg ggcaacctca cccacctgtc
   721 actcaagtac aacaacctca ctgtggtgcc ccgcaacctg ccttccagcc tggagtatct
   781 gctgttgtcc tacaaccgca tcgtcaaact ggcgcctgag gacctggcca atctgaccgc
   841 cctgcgtgtg ctcgatgtgg gcggaaattg ccgccgctgc gaccacgctc ccaacccctg
   901 catggagtgc cctcgtcact tcccccagct acatcccgat accttcagcc acctgagccg
   961 tcttgaaggc ctggtgttga aggacagttc tctctcctgg ctgaatgcca gttggttccg
   1021 tgggctggga aacctccgag tgctggacct gagtgagaac ttcctctaca aatgcatcac
   1081 taaaaccaag gccttccagg gcctaacaca gctgcgcaag cttaacctgt ccttcaatta
   1141 ccaaaagagg gtgtcctttg cccacctgtc tctggcccct tccttcggga gcctggtcgc
   1201 cctgaaggag ctggacatgc acggcatctt cttccgctca ctcgatgaga ccacgctccg
   1261 gccactggcc cgcctgccca tgctccagac tctgcgtctg cagatgaact tcatcaacca
   1321 ggcccagctc ggcatcttca gggccttccc tggcctgcgc tacgtggacc tgtcggacaa
   1381 ccgcatcagc ggagcttcgg agctgacagc caccatgggg gaggcagatg gaggggagaa
   1441 ggtctggctg cagcctgggg accttgctcc ggccccagtg gacactccca gctctgaaga
   1501 cttcaggccc aactgcagca ccctcaactt caccttggat ctgtcacgga acaacctggt
   1561 gaccgtgcag ccggagatgt ttgcccagct ctcgcacctg cagtgcctgc gcctgagcca
   1621 caactgcatc tcgcaggcag tcaatggctc ccagttcctg ccgctgaccg gtctgcaggt
   1681 gctagacctg tcccacaata agctggacct ctaccacgag cactcattca cggagctacc
   1741 acgactggag gccctggacc tcagctacaa cagccagccc tttggcatgc agggcgtggg
   1801 ccacaacttc agcttcgtgg ctcacctgcg caccctgcgc cacctcagcc tggcccacaa
   1861 caacatccac agccaagtgt cccagcagct ctgcagtacg tcgctgcggg ccctggactt
   1921 cagcggcaat gcactgggcc atatgtgggc cgagggagac ctctatctgc acttcttcca
   1981 aggcctgagc ggtttgatct ggctggactt gtcccagaac cgcctgcaca ccctcctgcc
   2041 ccaaaccctg cgcaacctcc ccaagagcct acaggtgctg cgtctccgtg acaattacct
   2101 ggccttcttt aagtggtgga gcctccactt cctgcccaaa ctggaagtcc tcgacctggc
   2161 aggaaaccag ctgaaggccc tgaccaatgg cagcctgcct gctggcaccc ggctccggag
   2221 gctggatgtc agctgcaaca gcatcagctt cgtggccccc ggcttctttt ccaaggccaa
   2281 ggagctgcga gagctcaacc ttagcgccaa cgccctcaag acagtggacc actcctggtt
   2341 tgggcccctg gcgagtgccc tgcaaatact agatgtaagc gccaaccctc tgcactgcgc
   2401 ctgtggggcg gcctttatgg acttcctgct ggaggtgcag gctgccgtgc ccggtctgcc
   2461 cagccgggtg aagtgtggca gtccgggcca gctccagggc ctcagcatct ttgcacagga
   2521 cctgcgcctc tgcctggatg aggccctctc ctgggactgt ttcgccctct cgctgctggc
   2581 tgtggctctg ggcctgggtg tgcccatgct gcatcacctc tgtggctggg acctctggta
   2641 ctgcttccac ctgtgcctgg cctggcttcc ctggcggggg cggcaaagtg ggcgagatga
   2701 ggatgccctg ccctacgatg ccttcgtggt cttcgacaaa acgcagagcg cagtggcaga
   2761 ctgggtgtac aacgagcttc gggggcagct ggaggagtgc cgtgggcgct gggcactccg
   2821 cctgtgcctg gaggaacgcg actggctgcc tggcaaaacc ctctttgaga acctgtgggc
   2881 ctcggtctat ggcagccgca agacgctgtt tgtgctggcc cacacggacc gggtcagtgg
   2941 tctcttgcgc gccagcttcc tgctggccca gcagcgcctg ctggaggacc gcaaggacgt
   3001 cgtggtgctg gtgatcctga gccctgacgg ccgccgctcc cgctacgtgc ggctgcgcca
   3061 gcgcctctgc cgccagagtg tcctcctctg gccccaccag cccagtggtc agcgcagctt
   3121 ctgggcccag ctgggcatgg ccctgaccag ggacaaccac cacttctata accggaactt
   3181 ctgccaggga cccacggccg aatagccgtg agccggaatc ctgcacggtg ccacctccac
   3241 actcacctca cctctgc
<210> 7
   <211> 3868
   <212> DNA
   <213> Homo sapiens
<220>
   <223> Transkript Variante A des TLR9-Gens
<400> 7
   1 ggaggtcttg tttccggaag atgttgcaag gctgtggtga aggcaggtgc agcctagcct
   61 cctgctcaag ctacaccctg gccctccacg catgaggccc tgcagaactc tggagatggt
   121 gcctacaagg gcagaaaagg acaagtcggc agccgctgtc ctgagggcac cagctgtggt
   181 gcaggagcca agacctgagg gtggaagtgt cctcttagaa tggggagtgc ccagcaaggt
   241 gtacccgcta ctggtgctat ccagaattcc catctctccc tgctctctgc ctgagctctg
   301 ggccttagct cctccctggg cttggtagag gacaggtgtg aggccctcat gggatgtagg
   361 ctgtctgaga ggggagtgga aagaggaagg ggtgaaggag ctgtctgcca tttgactatg
   421 caaatggcct ttgactcatg ggaccctgtc ctcctcactg ggggcagggt ggagtggagg
   481 gggagctact aggctggtat aaaaatctta cttcctctat tctctgagcc gctgctgccc
   541 ctgtgggaag ggacctcgag tgtgaagcat ccttccctgt agctgctgtc cagtctgccc
   601 gccagaccct ctggagaagc ccctgccccc cagcatgggt ttctgccgca gcgccctgca
   661 cccgctgtct ctcctggtgc aggccatcat gctggccatg accctggccc tgggtacctt
   721 gcctgccttc ctaccctgtg agctccagcc ccacggcctg gtgaactgca actggctgtt
   781 cctgaagtct gtgccccact tctccatggc agcaccccgt ggcaatgtca ccagcctttc
   841 cttgtcctcc aaccgcatcc accacctcca tgattctgac tttgcccacc tgcccagcct
   901 gcggcatctc aacctcaagt ggaactgccc gccggttggc ctcagcccca tgcacttccc
   961 ctgccacatg accatcgagc ccagcacctt cttggctgtg cccaccctgg aagagctaaa
   1021 cctgagctac aacaacatca tgactgtgcc tgcgctgccc aaatccctca tatccctgtc
   1081 cctcagccat accaacatcc tgatgctaga ctctgccagc ctcgccggcc tgcatgccct
   1141 gcgcttccta ttcatggacg gcaactgtta ttacaagaac ccctgcaggc aggcactgga
   1201 ggtggccccg ggtgccctcc ttggcctggg caacctcacc cacctgtcac tcaagtacaa
   1261 caacctcact gtggtgcccc gcaacctgcc ttccagcctg gagtatctgc tgttgtccta
   1321 caaccgcatc gtcaaactgg cgcctgagga cctggccaat ctgaccgccc tgcgtgtgct
   1381 cgatgtgggc ggaaattgcc gccgctgcga ccacgctccc aacccctgca tggagtgccc
   1441 tcgtcacttc ccccagctac atcccgatac cttcagccac ctgagccgtc ttgaaggcct
   1501 ggtgttgaag gacagttctc tctcctggct gaatgccagt tggttccgtg ggctgggaaa
   1561 cctccgagtg ctggacctga gtgagaactt cctctacaaa tgcatcacta aaaccaaggc
   1621 cttccagggc ctaacacagc tgcgcaagct taacctgtcc ttcaattacc aaaagagggt
   1681 gtcctttgcc cacctgtctc tggccccttc cttcgggagc ctggtcgccc tgaaggagct
   1741 ggacatgcac ggcatcttct tccgctcact cgatgagacc acgctccggc cactggcccg
   1801 cctgcccatg ctccagactc tgcgtctgca gatgaacttc atcaaccagg cccagctcgg
   1861 catcttcagg gccttccctg gcctgcgcta cgtggacctg tcggacaacc gcatcagcgg
   1921 agcttcggag ctgacagcca ccatggggga ggcagatgga ggggagaagg tctggctgca
   1981 gcctggggac cttgctccgg ccccagtgga cactcccagc tctgaagact tcaggcccaa
   2041 ctgcagcacc ctcaacttca ccttggatct gtcacggaac aacctggtga ccgtgcagcc
   2101 ggagatgttt gcccagctct cgcacctgca gtgcctgcgc ctgagccaca actgcatctc
   2161 gcaggcagtc aatggctccc agttcctgcc gctgaccggt ctgcaggtgc tagacctgtc
   2221 ccacaataag ctggacctct accacgagca ctcattcacg gagctaccac gactggaggc
   2281 cctggacctc agctacaaca gccagccctt tggcatgcag ggcgtgggcc acaacttcag
   2341 cttcgtggct cacctgcgca ccctgcgcca cctcagcctg gcccacaaca acatccacag
   2401 ccaagtgtcc cagcagctct gcagtacgtc gctgcgggcc ctggacttca gcggcaatgc
   2461 actgggccat atgtgggccg agggagacct ctatctgcac ttcttccaag gcctgagcgg
   2521 tttgatctgg ctggacttgt cccagaaccg cctgcacacc ctcctgcccc aaaccctgcg
   2581 caacctcccc aagagcctac aggtgctgcg tctccgtgac aattacctgg ccttctttaa
   2641 gtggtggagc ctccacttcc tgcccaaact ggaagtcctc gacctggcag gaaaccagct
   2701 gaaggccctg accaatggca gcctgcctgc tggcacccgg ctccggaggc tggatgtcag
   2761 ctgcaacagc atcagcttcg tggcccccgg cttcttttcc aaggccaagg agctgcgaga
   2821 gctcaacctt agcgccaacg ccctcaagac agtggaccac tcctggtttg ggcccctggc
   2881 gagtgccctg caaatactag atgtaagcgc caaccctctg cactgcgcct gtggggcggc
   2941 ctttatggac ttcctgctgg aggtgcaggc tgccgtgccc ggtctgccca gccgggtgaa
   3001 gtgtggcagt ccgggccagc tccagggcct cagcatcttt gcacaggacc tgcgcctctg
   3061 cctggatgag gccctctcct gggactgttt cgccctctcg ctgctggctg tggctctggg
   3121 cctgggtgtg cccatgctgc atcacctctg tggctgggac ctctggtact gcttccacct
   3181 gtgcctggcc tggcttccct ggcgggggcg gcaaagtggg cgagatgagg atgccctgcc
   3241 ctacgatgcc ttcgtggtct tcgacaaaac gcagagcgca gtggcagact gggtgtacaa
   3301 cgagcttcgg gggcagctgg aggagtgccg tgggcgctgg gcactccgcc tgtgcctgga
   3361 ggaacgcgac tggctgcctg gcaaaaccct ctttgagaac ctgtgggcct cggtctatgg
   3421 cagccgcaag acgctgtttg tgctggccca cacggaccgg gtcagtggtc tcttgcgcgc
   3481 cagcttcctg ctggcccagc agcgcctgct ggaggaccgc aaggacgtcg tggtgctggt
   3541 gatcctgagc cctgacggcc gccgctcccg ctatgtgcgg ctgcgccagc gcctctgccg
   3601 ccagagtgtc ctcctctggc cccaccagcc cagtggtcag cgcagcttct gggcccagct
   3661 gggcatggcc ctgaccaggg acaaccacca cttctataac cggaacttct gccagggacc
   3721 cacggccgaa tagccgtgag ccggaatcct gcacggtgcc acctccacac tcacctcacc
   3781 tctgcctgcc tggtctgacc ctcccctgct cgcctccctc accccacacc tgacacagag
   3841 caggcactca ataaatgcta ccgaaggc
<210> 8
   <211> 3110
   <212> DNA
   <213> Homo sapiens
<220>
   <223> Transkript Variante B des TLR9-Gens
<400> 8
   1 tggtgaactg caactggctg ttcctgaagt ctgtgcccca cttctccatg gcagcacccc
   61 gtggcaatgt caccagcctt tccttgtcct ccaaccgcat ccaccacctc catgattctg
   121 actttgccca cctgcccagc ctgcggcatc tcaacctcaa gtggaactgc ccgccggttg
   181 gcctcagccc catgcacttc ccctgccaca tgaccatcga gcccagcacc ttcttggctg
   241 tgcccaccct ggaagagcta aacctgagct acaacaacat catgactgtg cctgcgctgc
   301 ccaaatccct catatccctg tccctcagcc ataccaacat cctgatgcta gactctgcca
   361 gcctcgccgg cctgcatgcc ctgcgcttcc tattcatgga cggcaactgt tattacaaga
   421 acccctgcag gcaggcactg gaggtggccc cgggtgccct ccttggcctg ggcaacctca
   481 cccacctgtc actcaagtac aacaacctca ctgtggtgcc ccgcaacctg ccttccagcc
   541 tggagtatct gctgttgtcc tacaaccgca tcgtcaaact ggcgcctgag gacctggcca
   601 atctgaccgc cctgcgtgtg ctcgatgtgg gcggaaattg ccgccgctgc gaccacgctc
   661 ccaacccctg catggagtgc cctcgtcact tcccccagct acatcccgat accttcagcc
   721 acctgagccg tcttgaaggc ctggtgttga aggacagttc tctctcctgg ctgaatgcca
   781 gttggttccg tgggctggga aacctccgag tgctggacct gagtgagaac ttcctctaca
   841 aatgcatcac taaaaccaag gccttccagg gcctaacaca gctgcgcaag cttaacctgt
   901 ccttcaatta ccaaaagagg gtgtcctttg cccacctgtc tctggcccct tccttcggga
   961 gcctggtcgc cctgaaggag ctggacatgc acggcatctt cttccgctca ctcgatgaga
   1021 ccacgctccg gccactggcc cgcctgccca tgctccagac tctgcgtctg cagatgaact
   1081 tcatcaacca ggcccagctc ggcatcttca gggccttccc tggcctgcgc tacgtggacc
   1141 tgtcggacaa ccgcatcagc ggagcttcgg agctgacagc caccatgggg gaggcagatg
   1201 gaggggagaa ggtctggctg cagcctgggg accttgctcc ggccccagtg gacactccca
   1261 gctctgaaga cttcaggccc aactgcagca ccctcaactt caccttggat ctgtcacgga
   1321 acaacctggt gaccgtgcag ccggagatgt ttgcccagct ctcgcacctg cagtgcctgc
   1381 gcctgagcca caactgcatc tcgcaggcag tcaatggctc ccagttcctg ccgctgaccg
   1441 gtctgcaggt gctagacctg tcccacaata agctggacct ctaccacgag cactcattca
   1501 cggagctacc acgactggag gccctggacc tcagctacaa cagccagccc tttggcatgc
   1561 agggcgtggg ccacaacttc agcttcgtgg ctcacctgcg caccctgcgc cacctcagcc
   1621 tggcccacaa caacatccac agccaagtgt cccagcagct ctgcagtacg tcgctgcggg
   1681 ccctggactt cagcggcaat gcactgggcc atatgtgggc cgagggagac ctctatctgc
   1741 acttcttcca aggcctgagc ggtttgatct ggctggactt gtcccagaac cgcctgcaca
   1801 ccctcctgcc ccaaaccctg cgcaacctcc ccaagagcct acaggtgctg cgtctccgtg
   1861 acaattacct ggccttcttt aagtggtgga gcctccactt cctgcccaaa ctggaagtcc
   1921 tcgacctggc aggaaaccag ctgaaggccc tgaccaatgg cagcctgcct gctggcaccc
   1981 ggctccggag gctggatgtc agctgcaaca gcatcagctt cgtggccccc ggcttctttt
   2041 ccaaggccaa ggagctgcga gagctcaacc ttagcgccaa cgccctcaag acagtggacc
   2101 actcctggtt tgggcccctg gcgagtgccc tgcaaatact agatgtaagc gccaaccctc
   2161 tgcactgcgc ctgtggggcg gcctttatgg acttcctgct ggaggtgcag gctgccgtgc
   2221 ccggtctgcc cagccgggtg aagtgtggca gtccgggcca gctccagggc ctcagcatct
   2281 ttgcacagga cctgcgcctc tgcctggatg aggccctctc ctgggactgt ttcgccctct
   2341 cgctgctggc tgtggctctg ggcctgggtg tgcccatgct gcatcacctc tgtggctggg
   2401 acctctggta ctgcttccac ctgtgcctgg cctggcttcc ctggcggggg cggcaaagtg
   2461 ggcgagatga ggatgccctg ccctacgatg ccttcgtggt cttcgacaaa acgcagagcg
   2521 cagtggcaga ctgggtgtac aacgagcttc gggggcagct ggaggagtgc cgtgggcgct
   2581 gggcactccg cctgtgcctg gaggaacgcg actggctgcc tggcaaaacc ctctttgaga
   2641 acctgtgggc ctcggtctat ggcagccgca agacgctgtt tgtgctggcc cacacggacc
   2701 gggtcagtgg tctcttgcgc gccagcttcc tgctggccca gcagcgcctg ctggaggacc
   2761 gcaaggacgt cgtggtgctg gtgatcctga gccctgacgg ccgccgctcc cgctatgtgc
   2821 ggctgcgcca gcgcctctgc cgccagagtg tcctcctctg gccccaccag cccagtggtc
   2881 agcgcagctt ctgggcccag ctgggcatgg ccctgaccag ggacaaccac cacttctata
   2941 accggaactt ctgccaggga cccacggccg aatagccgtg agccggaatc ctgcacggtg
   3001 ccacctccac actcacctca cctctgcctg cctggtctga ccctcccctg ctcgcctccc
   3061 tcaccccaca cctgacacag agcaggcact caataaatgc taccgaaggc